# EUROPÄISCHE PATENTSCHRIFT

(11) **EP 0 529 107 B1**
(45) Veröffentlichungstag und Bekanntmachung des Hinweises auf die Patenterteilung: **01.02.1995**
(21) Anmeldenummer: 91112082.2
(22) Anmeldetag: 19.07.1991
(51) Int. Cl.: C11B 3/00, C11B 7/00

(54) **Verfahren zur Gewinnung eines Öls mit einstellbarem Carotingehalt aus Palmöl**
Process for obtaining an oil with adjustable carotene content from palm oil
Procédé d'obtention d'une huile à teneur en carotène reglée à partir de l'huile de palme

(43) Veröffentlichungstag der Anmeldung: 03.03.1993
(73) Patentinhaber: DEUTSCHE GESELLSCHAFT FÜR TECHNISCHE ZUSAMMENARBEIT( GTZ) GmbH, 65760 Eschborn (DE)
(72) Erfinder: Plonis, Gerhard Fred, 13 083 Campinas, SP (BR); Trujillo-Quijano, José, 13081 Campinas S.P. (BR)
(74) Vertreter: Gudel, Diether

(56) Entgegenhaltungen:
- GB-A- 1 425 053
- GB-A- 1 510 056
- GB-A- 2 144 143
- US-A- 2 416 146

## Beschreibung

Die Erfindung betrifft ein Verfahren zur Gewinnung eines Oleins mit einem Gehalt von 750 - 1.000 ppm Carotin aus rohem Palmöl.

Es wurde schon seit langer Zeit versucht, das Carotin des Palmöls zum Färben von Margarine und anderen Nahrungsmitteln einzusetzen. Verschiedene Anreicherungsverfahren wurden benutzt, aber der Erfolg scheiterte immer an zu hohen Kosten oder an dem Eigengeschmack des carotinhaltigen Öls. Ferner war bei dem Einsatz von Palmöl immer der unterschiedliche Gehalt an Carotin in dem angereicherten Produkt oder dem natürlichen Palmöl ein Hindernis für den Einsatz als Lebensmittelfarbe.

Der Eigengeschmack kann auch nicht durch die modernen Raffinationsmethoden entfernt werden, weil bei den hohen Temperaturen das Carotin entweder völlig oder teilweise zerstört wird. Deshalb wird in der Industrie zum Färben synthetisches Carotin eingesetzt. Dieses wird in einem Raffinat zu einer konzentrierten Lösung mit bekanntem Gehalt gelöst und dann über Dosieranlagen der Produktmischung in der notwendigen Konzentration zugesetzt.

Die GB-A-2,144,143 beschreibt ein Verfahren zum Reinigen von Palmöl, bei dem das rohe Palmöl gebleicht und entschleimt wird.

Hier wird beschrieben, daß bei der Flüssig-flüssig-Extraktion von Palmöl mit Ethanol ein Raffinat erhalten wird, das einen hohen Carotinanteil aufweist, geruchlos ist und einen niedrigen Anteil an freien Fettsäuren besitzt und daher als natürlicher Farbstoff eingesetzt werden kann.

Die GB-A-1,425,053 beschreibt ein Verfahren zum Raffinieren von Öl, wobei die gefärbten Anteile des Öls mit entfernt werden. Bei diesem Verfahren wird auch Citronensäure eingesetzt.

Die Aufgabe der vorliegenden Erfindung besteht darin, ein besseres Verfahren zur Verfügung zu stellen, als dies in der GB-A-2,144,143 beschrieben ist.

Diese Aufgabe wird durch die Merkmale von Patentanspruch 1 gelöst.

Bei Versuchen des Erfinders, aus dem Palmöl sowohl die freien Fettsäuren als auch die Monoglyzeride und einen Teil der Diglyzeride zu entfernen, ohne den Carotingehalt nennenswert zu mindern, konnte mit wasserhaltigen niederen Alkoholen und Ketonen mit Hilfe der Flüssig-flüssig-Extraktion sehr gute Ergebnisse erzielt werden. Die Zielgruppe bei diesen Versuchen waren die Diglyzeride, die bei der Fraktionierung von Palmöl bekanntlich sehr negative Effekte hervorrufen. Es konnte der ffa-Gehalt auf unter 0,1 % gesenkt werden, die Monoglyzeride im extrahierten Palmöl waren mit der TLC nicht mehr nachweisbar und die Diglyzeride waren auf ca. 1/3 des ursprünglichen Gehalts reduziert.

Überraschend stellte sich heraus, daß auch die oxidierten Glyzeride wie auch die Geschmacks- und Geruchsstoffe des Palmöls auf einen organoleptisch nicht mehr nachweisbaren Gehalt reduziert wurden. Ähnliche Ergebnisse wurden auch mit anderen Ölen erzielt.

Genauso wurde aus dem Carotingemisch besonders die Isomeren gelöst, so daß auf Gesamtcarotin berechnet der Anteil an α- und β-Carotin, und damit der Gehalt an Provitamin A, anstieg.

Großes Augenmerk wurde auf die Eigenschaften des Extraktionsmittels gelegt, besonders im Hinblick auf die unvermeidlichen Spuren, die bei sorgfältigster Entfernung des Lösungsmittels im Öl verbleiben. Als bestes Lösungsmittel stellte sich der Ethylalkohol heraus, der eine niedrige Dichte sowie eine niedrige Viskosität und auch einen niedrigen Siedepunkt aufweist. Da er als Genußmittel dient, sind die Restspuren von weniger als 0,1% im Öl auch nicht als bedenklich anzusehen.

Methanol scheidet wegen seiner Giftigkeit aus und die Propanole haben bereits einen recht hohen Siedepunkt und auch höhere Viskositäten. Ketone sind nicht für den Einsatz in Lebensmitteln zu empfehlen.

Der Wassergehalt kann in weiten Grenzen schwanken. Aus wirtschaftlichen Gründen sollte der Wassergehalt aber so niedrig wie möglich sein, um eine gute Löslichkeit der zu entfernenden Stoffe im Alkohol zu gewährleisten und auf der anderen Seite so hoch, daß Umesterungen nicht stattfinden.

Nach Entfernen des Alkohols hat man ein Palmöl mit hervorragenden Eigenschaften zur Trockenfraktionierung in ein oder zwei Stufen. Dabei kann der Gehalt an Carotinoiden erhöht werden, da die Kristalle praktisch frei von diesen sind und die Carotinoide im Olein verbleiben. Durch diese Eigenschaft kann der Carotinoidgehalt sehr genau im verbleibenden Olein eingestellt werden. Dieses kann über ein- oder mehrstufige Fraktionierung gut gesteuert werden.

### Beispiel 1

4 kg rohes Palmöl mit einer ffa von 3,65% wurde totalentschleimt, um den Phosphatidgehalt abzusenken. Dieses Öl wurde in einer Extraktionskolonne mit einem Radius von r = 15 mm und einer Höhe h = 1.500 mm extrahiert. Die Extraktionskolonne war mit drei Füllkörperschichten mit einer Höhe von je 300 mm aus Wilsonspiralen 3 mm ⌀ gefüllt. Die Extraktionstemperatur lag bei 50°C und die Ölmenge betrug 250 ml/h bei einem Lösungsmittelfluß von 500 ml/h. Dabei wurden folgende Ergebnisse erzielt:

| Analysenwert | Rohöl | Extrakt | extrah. Öl |
|---|---|---|---|
| ffa (%) | 3,65 | 22,8 | 0,08 |
| Diglyzeride (%) | 4,6 | 20,2 | 1,7 |
| Monoglyzeride + Polare (%) | 2,3 | 5,4 | 0,2 |
| Triglyzeride (%) | 90,6 | 45,8 | 97,9 |
| Carotingehalt (ppm) | 590 | 305 | 650 |
| Ausbeute (%) | 100 | 14 | 86 |

Dieses Raffinat wurde nach Entfernen des Alkohols in einer Trockenfraktionierung bis auf eine Temperatur von 20°C gekühlt und filtriert. Das erhaltene Olein hatte einen CP von 7°C, JZ von 58,3 und einen Carotinoidgehalt von 800 ppm, wobei 80% aus α- und β-Carotin bestand.

### Beispiel 2

4 kg rohes Palmöl mit einer ffa von 8,8% wurde totalentschleimt, um den Phosphatidgehalt abzusenken. Dieses Öl wurde wurde in der oben beschrieben Extraktionskolonne extragiert. Die Extraktionstemperatur lag bei 50% und die Ölmenge betrug 200 ml/h bei einem Lösungsmittelfluß von 400 ml/h. Dabei wurden folgende Ergebnisse erzielt:

| Analysenwert | Rohöl | Extrakt | extrah. Öl |
|---|---|---|---|
| ffa (%) | 8,6 | 40,6 | 0,1 |
| Carotingehalt (ppm) | 560 | 320 | 620 |
| Gehalt an α- und β-Carotin (ppm) | 420 | 160 | 500 |
| Ausbeute % | 100 | 21 | 79 |

Dieses Raffinat wurde nach Entfernen des Alkohols in einer Trockenfraktionierung bis auf eine Temperatur von 24°C gekühlt und filtriert (Stufe 1). Daran schloß sich eine zweite Fraktionierung bei 20°C an (Stufe 2). Folgende Produkte wurden dabei erhalten:

| Analysenwert | Stufe 1 | Stufe 2 | Olein |
|---|---|---|---|
| ffa (%) | 0,1 | 0,1 | 0,1 |
| Carotingehalt | 70 | 100 | 850 |
| Gehalt α- und β-Carotin (ppm) | 56 | 81 | 690 |
| Ausbeute (%) | 19 | 35 | 46 |

Erfindungsgemäß wird das Palmöl auch totalentschleimt, d.h. es wird der Phosphorgehalt auf unter 2 ppm gesenkt. Hierzu die folgenden Überlegungen:
Nach der Fällung der Phosphatide mit Wasser enthalten die meisten Öle noch nennenswerte Mengen an Phosphatiden, die nicht im Wasser quellbar sind. Diese stören die Entsäuerung, da sie die Emulsionsbildung fördern sowie Triglyzeride einschließen und damit die Ausbeute an Raffinat mindern.

Die Nachentschleimung vor der Raffination dient der Verbesserung der Endausbeute und sollte bei möglichst niedrigen Temperaturen durchgeführt werden. Bei diesen Temperaturen ist jedoch das Öl sehr viskos und die Filtration wird erschwert.

Der Alkohol hat auf die Phosphatide zusätzlich eine fällende Wirkung.

### Beispiel 3

1 kg Palmöl wurde auf 45°C unter Vakuum erhitzt und nach Brechen des Vakuums unter Rühren mit 100 ml einer alkoholischen Lösung von Citronensäure versetzt. Darauf wurden 10 g Bleicherde zugegeben und 30 min gerührt.

Danach wurde das Gemisch durch Filtration von der Bleicherde und den ausgefallenen Phosphatiden befreit.

| | |
|---|---|
| P-Gehalt vor Behandlung | 12 ppm |
| P-Gehalt nach Behandlung | 1 ppm. |

### Beispiel 4

1 kg Sojaöl wurde auf 35°C unter Vakuum erhitzt und nach Brechen des Vakuums unter Rühren mit 100 ml einer alkoholischen Lösung von Citronensäure versetzt. Darauf wurden 10 g Bleicherde zugegeben und 30 min gerührt.

Danach wurde das Gemisch durch Filtration von der Bleicherde und den ausgefallenen Phisphatiden befreit.

| | |
|---|---|
| P-Gehalt vor Behandlung | 25 ppm, |
| P-Gehalt nach Behandlung | 2 ppm. |

Die vorstehend verwendeten Ausdrücke haben folgende Bedeutung:
- ffa-Gehalt:: Gehalt an freien Fettsäuren (free fatty acids),
- TLC:: Dünnschicht-Chromatographie (Thin layer Chromatography),
- Wilsonspirale:: Einsatz in einer Fraktionierungskolonne,
- CP:: Trübungspunkt (Cloud point),
- JZ:: Jodzahl.

## Patentansprüche

1. Verfahren zur Gewinnung eines Oleins mit einem Gehalt von 750 - 1000 ppm Carotin aus rohem Palmöl,
**dadurch gekennzeichnet**,
daß man das Palmöl
a) zuerst einer Totalentschleimung unterzieht, wozu das trockene Öl mit einer Temperatur von 20 - 50° C
a1) mit 8 - 10% Ethanol mit einem Gehalt von 75 - 96% Enthanol versetzt wird, in dem 1% Citronensäure gelöst sind und
a2) mit Filtererde versetzt wird, worauf
a3) abfiltriert wird
b) daß man dieses entschleimte Palmöl einer Flüssig-flüssig-Extraktion unterzieht und
c) dieses so gereinigte Palmöl einer ein- oder mehrstufigen Trockenfraktionierung unterwirft, bei der das Olein mit einem vorbestimmten Gehalt an Carotin anfällt.

2. Verfahren nach Anspruch 1,
**dadurch gekennzeichnet,**
daß man als Extraktionsmittel kurzkettige Alkohole oder Ketone verwendet.

3. Verfahren nach Anspruch 2,
**dadurch gekennzeichnet,**
daß man als Extraktionsmittel Ethanol verwendet.

4. Verfahren nach Anspruch 3,
**dadurch gekennzeichnet,**
daß das Extraktionsmittel 1 - 25 Vol.% Wasser enthält.

5. Verfahren nach Anspruch 4,
**dadurch gekennzeichnet,**
daß das Volumenverhältnis Öl/Lösungsmittel 1/0,5 bis 1/4 ist.

6. Verfahren nach Anspruch 5,
**dadurch gekennzeichnet,**
daß als Extraktionstemperaturen 20 - 60°C benutzt werden.

## Claims

1. A process for obtaining an olein with a carotene content of 750 - 1000 ppm from crude palm oil, **characterised in that**
a) the palm oil is firstly completely de-gummed, to which end the dry oil, at a temperature of 20 - 50°C:
a1) is mixed with 8 - 10% ethanol with an ethanol content of 75 - 96%, in which 1% citric acid has been dissolved, and
a2) is mixed with filter earth, and
a3) is subsequently filtered off,
b) the de-gummed palm oil thus obtained is subjected to liquid-liquid extraction, and
c) the palm oil purified in this manner undergoes single-stage or multi-stage dry fractionation, in which the olein is produced with a pre-determined carotene content.

2. A process in accordance with claim 1, **characterised in that** short-chain alcohols or ketones are used as extraction agents.

3. A process in accordance with claim 2, **characterised in that** ethanol is used as an extraction agent.

4. A process in accordance with claim 3, **characterised in that** the extraction agent contains 1 - 25 % by volume of water.

5. A process in accordance with claim 4, **characterised in that** the oil/solvent volume ratio is 1:0.5 to 1:4.

6. A process in accordance with claim 5, **characterised in that** the extraction temperatures are 20 - 60°C.

## Revendications

1. Procédé d'obtention d'une oléine d'une teneur de 750-1000 ppm de carotène à partir d'huile de palme brute, caractérisé en ce que:
a) on soumet tout d'abord l'huile de palme à un dégommage total au cours duquel on mélange l'huile séchée à une température de 20-50°C,
a1) à 8 à 10% d'éthanol ayant une teneur en éthanol de 75 à 95%, dans lequel on a dissous 1% d'acide citrique,
a2) on la mélange avec une terre filtrante, après quoi
a3) on sépare par filtration,
b) on soumet cette huile de palme dégommée à une extraction liquide-liquide, et
c) on soumet cette huile ainsi épurée à un fractionnement à sec en un ou plusieurs stades, au cours duquel l'oléine est produite avec une teneur en carotène prédéterminée.

2. Procédé selon la revendication 1, caractérisé en ce qu'on utilise comme milieux d'extraction des alcools ou des cétones à chaînes courtes.

3. Procédé selon la revendication 2, caractérisé en ce qu'on utilise comme milieu d'extraction de l'éthanol.

4. Procédé selon la revendication 3, caractérisé en ce que le milieu d'extraction contient 1 à 25 volumes d'eau pour-cent.

5. Procédé selon la revendication 4, caractérisé en ce que le rapport volumique huile/solvant est de 1/0,5 à 1/4.

6. Procédé selon la revendication 5, caractérisé en ce qu'on utilise comme températures d'extraction des températures de 20 à 60°C.
